# EUROPEAN PATENT APPLICATION

(11) **EP 4 835 489 A1**
(43) Date of publication of application: **04.11.2026**
(21) Application number: 26164534.5
(22) Date of filing: 13.03.2026
(51) Int. Cl.: G01N 35/10, C12M 1/26

(54) **SAMPLING APPARATUS, SAMPLING METHOD, AND SAMPLING PROGRAM**

(30) Priority: 19.03.2025 JP 2025045749
(71) Applicant: Yokogawa Electric Corporation, Musashino-shi, Tokyo 180-8750 (JP)
(72) Inventor: NISHIDE, Kohki, Musashino-shi, Tokyo 180-8750 (JP)
(74) Representative: Winter, Brandl - Partnerschaft mbB

(57) **Abstract**

A sampling apparatus (10) is a sampling apparatus that takes a sample from a designated sampling position of an analysis target by using a tip. The sampling apparatus (10) acquires measurement data including positional information on each of the tips in use, and calculates positional deviation that is an error with respect to the sampling position of each of the tips based on the acquired measurement data.

## Description

### FIELD

The present disclosure is related to a sampling apparatus, a sampling method, and a sampling program.

### BACKGROUND

A technology for taking, in a fully automatic manner, a sample from a specific site or a specific region (appropriately referred to as a "sampling position") at a single-cell level by using a tip that is stored in a tip rack while imaging cells in culture is known (see, for example, Japanese Laid-open Patent Publication No. 2023-018912).

However, it is difficult to improve sampling efficiency. For example, in the technology as described above, a suction unit or the like for sucking a tip at the time of sampling may naturally move over time, so that an error (appropriately referred to as "positional deviation") form a designated sampling position may occur.

The present disclosure has been conceived in view of the foregoing situations, and an object of the present disclosure is to improve sampling efficiency.

### SUMMARY OF THE INVENTION

According to an aspect of the embodiments, a sampling apparatus that takes a sample from a designated sampling position of an analysis target by using a tip, the sampling apparatus includes a processor that acquires positional information on each of tips in use, and calculates an error with respect to the sampling position of each of the tips based on the acquired positional information.

According to an aspect of the embodiments, a sampling method implemented by a sampling apparatus that takes a sample from a designated sampling position of an analysis target by using a tip, the sampling method includes acquiring positional information on each of the tips in use and calculating an error with respect to the sampling position of each of the tips based on the acquired positional information.

According to an aspect of the embodiments, a sampling program that causes a sampling apparatus that takes a sample from a designated sampling position of an analysis target by using a tip to execute a process, the process includes acquiring positional information on each of the tips in use, and calculating an error with respect to the sampling position of each of the tips based on the acquired positional information.

### BRIEF DESCRIPTION OF THE DRAWINGS

FIG. 1 is a diagram illustrating a configuration example and a process example of a sampling system according to one embodiment;
FIG. 2 is a diagram for explaining a sampling system according to a reference technology;
FIG. 3 is a block diagram illustrating a configuration example of each of apparatuses in the sampling system according to one embodiment;
FIG. 4 is a diagram illustrating an example of a measurement data storage unit of the sampling apparatus according to one embodiment;
FIG. 5 is a diagram illustrating an example of a calculation result storage unit of the sampling apparatus according to one embodiment;
FIG. 6 is a diagram illustrating an example of a correction model storage unit of the sampling apparatus according to one embodiment;
FIG. 7 is a diagram illustrating a specific example 1 of measurement data according to one embodiment;
FIG. 8 is a diagram illustrating a specific example 2 of the measurement data according to one embodiment;
FIG. 9 is a diagram illustrating a specific example 3 of the measurement data according to one embodiment;
FIG. 10 is a flowchart illustrating an example of the flow of the entire process of the sampling system according to one embodiment;
FIG. 11 is a flowchart illustrating an example of the flow of an acquisition control process performed by the sampling system according to one embodiment;
FIG. 12 is a flowchart illustrating an example of the flow of a training control process performed by the sampling system according to one embodiment;
FIG. 13 is a flowchart illustrating an example of the flow of a calculation control process performed by the sampling system according to one embodiment;
FIG. 14 is a flowchart illustrating an example of the flow of a sampling control process performed by the sampling system according to one embodiment; and
FIG. 15 is a diagram illustrating a hardware configuration example according to one embodiment.

### DETAILED DESCRIPTION OF THE PREFERRED EMBODIMENTS

Embodiments of a sampling apparatus, sampling method, and a sampling program according to the present disclosure will be described in detail below with reference to the drawings. Meanwhile, the present disclosure is not limited by the embodiments described below.

A configuration and a process of a sampling system 100 according to one embodiment, a configuration and a process of each of apparatuses of the sampling system 100, a specific example of each of processes of the sampling system 100, a flow of each of the processes of the sampling system 100, and effects of one embodiment will be described below.

### 1. Configuration and process of sampling system 100

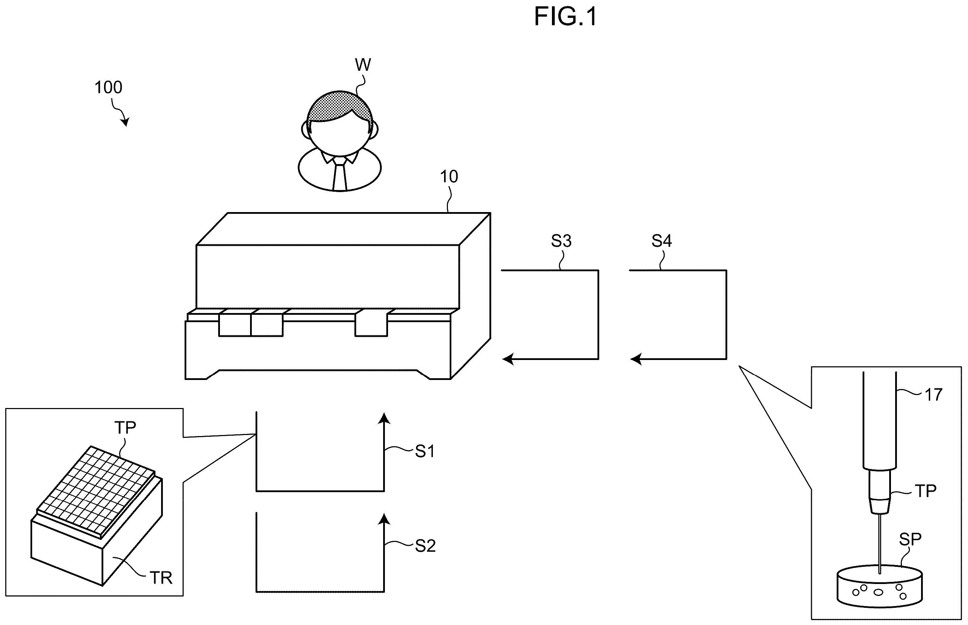

A configuration and a process of the sampling system 100 according to one embodiment will be described below with reference to FIG. 1. FIG. 1 is a diagram illustrating a configuration example and a process example of the sampling system 100 according to one embodiment. In the following, a configuration example of the entire sampling system 100, a process example of the entire sampling system 100, and effects of the sampling system 100 will be described.

Meanwhile, in one embodiment, a cell in culture will be described as one example of an analysis target, but types of the analysis target and fields of application are not specifically limited. Further, in the following descriptions, a term "cell" may include not only a single cell, but also a spheroid, a histological section, and the like.

### 1-1. Configuration example of entire sampling system 100

A configuration example of the entire sampling system 100 will be described. As illustrated in FIG. 1, the sampling system 100 includes a sampling apparatus 10. The sampling apparatus 10 is communicably connected in a wired or wireless manner via a predetermined communication network (not illustrated). Meanwhile, various kinds of communication networks, such as the Internet or a dedicated line, may be adopted as the predetermined communication network.

### 1-1-1. Sampling apparatus 10

The sampling apparatus 10 is a sampling apparatus that is used by a worker W who is an administrator of a cell culture facility. For example, the sampling apparatus 10 is installed in the cell culture facility and operated by the worker W. Meanwhile, the sampling system 100 illustrated in FIG. 1 may include the plurality of sampling apparatuses 10.

In the following, a series of processes of attaching the tip TP on the tip rack TR, puncturing the analysis target with the tip TP, sucking a sample SP, storing the sucked sample SP, and ejecting the sucked sample SP to a storage container will be described as "sampling". Further, a position at which the sample SP is taken in an analysis target designated by the worker W will be described as a "sampling position P₀", and a position at which a leading end of the tip TP is actually inserted will be described as a "leading end position Pt". Furthermore, an error between the sampling position P₀ and the leading end position Pₜ will be described as "positional deviation ΔP₀". Moreover, a simple error between a designated movement path and the leading end position Pₜ will be described as "positional deviation ΔP".

### 1-2. Process example of entire sampling system 100

A process example of the entire sampling system 100 will be described below with reference to FIG. 1. As illustrated in FIG. 1, the sampling system 100 performs processes from Step S1 to Step S4. In the following, as a process example of the entire sampling system 100, a measurement data acquisition process (Step S1), a model training process (Step S2), a calculation result output process (Step S3), and a sampling process (Step S4) will be described. Meanwhile, the processes from Step S1 to Step S4 below may be performed in different order. Further, some of the processes from Step S1 to Step S4 below may be omitted.

### 1-2-1. Measurement data acquisition process

Firstly, the sampling apparatus 10 performs the measurement data acquisition process (Step S1). For example, the sampling apparatus 10 acquires measurement data D that includes positional information on each of the tips TP in use. In this case, the sampling apparatus 10 acquires the measurement data D that includes positional information on each of the storage positions Pₛ in the tip rack TR in which each of the tips TP is stored. Furthermore, the sampling apparatus 10 may acquire the measurement data D at the time of actually taking the sample SP, or may acquire the measurement data D at the time of trial operation in which the sample SP is not taken.

Here, the measurement data D is image data D_{I} that includes an image I in which a leading end of each of the tips TP in use is captured, and indicates time series data of positional information from the start of use to sampling of the sample SP, for example, from the time of attachment of the tip TP to the time of puncture with the tip TP. Further, the measurement data D is temperature data D_{T} that is obtained by measuring temperature T of each of the tips TP in use, and indicates time series data of the temperature T from the start of use to sampling of the sample SP, for example, from the time of attachment of the tip TP to the time of puncture with the tip TP.

Furthermore, the sampling apparatus 10 stores the acquired measurement data D as time series data of coordinates on a plane indicating the positional deviation ΔP of each of the storage positions Pₛ in the tip rack TR (for example: positional deviation Δx in an X direction and positional deviation Δy in a Y direction). Moreover, the sampling apparatus 10 stores the acquired measurement data D, together with the time series data of the positional deviation ΔP, as the time series data of the temperature data D_{T}.

### 1-2-2. Model training process

Secondly, the sampling apparatus 10 performs the model training process (Step S2). For example, the sampling apparatus 10 inputs, as training data, the acquired measurement data D to a correction model CM that is a machine learning model, and trains the correction model CM. In this case, for example, the sampling apparatus 10 trains the correction model CM so as to output the positional deviation ΔP₀ at the time of sampling of the sample SP in accordance with input of a storage position Pₛ in the tip rack TR and the temperature T of the tip TP at the start of use.

### 1-2-3. Calculation result output process

Thirdly, the sampling apparatus 10 performs the calculation result output process (Step S3). For example, the sampling apparatus 10, at the start of sampling of the sample SP, identifies each of the storage positions Pₛ in the tip rack TR, the temperature T, and a time t1 at which the tip TP is attached, inputs the identified information to the correction model CM, and calculates the positional deviation ΔP₀ at a time t2 at which puncture is performed with each of the tips TP. Further, the sampling apparatus 10, by applying a linear interpolation method to the time series data of the positional deviation ΔP indicated by the measurement data D, may calculate the positional deviation ΔP₀ of each of the tips TP at the time of puncture, without using the correction model CM.

In this case, the sampling apparatus 10 is able to calculate, as a calculation result CR, the positional deviation ΔP in which the temperature T and a temporal change are taken into account, such as "a tip TP at the storage position Pₛ of A1 in the tip rack TR moves 5 micrometers (µm) in a southeast direction after a lapse of 10 seconds in an environment in which temperature is 37°C".

### 1-2-4. Sampling process

Fourthly, the sampling apparatus 10 performs the sampling process (Step S4). For example, the sampling apparatus 10 refers to the calculated positional deviation ΔP₀ of each of the tips TP, corrects the leading end position Pₜ of the tip TP at the time of puncture with the tip TP, punctures a cell with the tip TP, sucks the sample SP, and ejects the sample SP to a storage container to perform sampling of the sample SP.

### 1-3. Effects of sampling system 100

In the following, problems with a sampling system 100-P according to a reference technology will be first described, and thereafter, an overview and effects of the sampling system 100 will be described.

### 1-3-1. Problems with sampling system 100-P

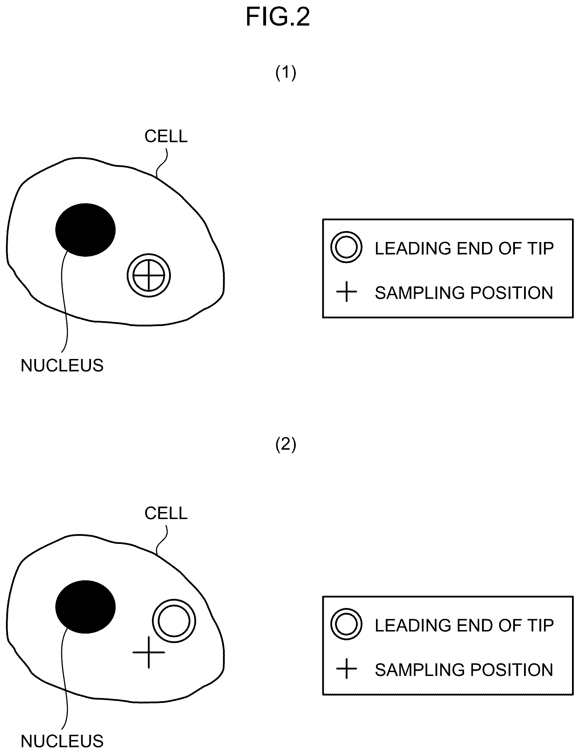

Problems with the sampling system 100-P according to the reference technology will be described below with reference to FIG. 2. FIG. 2 is a diagram for explaining the sampling system 100-P according to the reference technology. For example, the sampling system 100-P stains a nucleus in a cell, inserts the tip TP at the designated sampling position P₀ around the nucleus, sucks the sample SP, and ejects the sample SP to the storage container to take the sample SP. In the example illustrated in FIG. 2(1), the positional deviation ΔP₀ has not occurred between the designated sampling position P₀ and the leading end position Pₜ of the tip TP. In contrast, in the sampling system 100-P, due to slight deformation caused by vibration V or the temperature T, residual strass at the time of attachment of the tip TP, an installation environment, or the like, a suction unit or the like for sucking the tip TP may naturally moves over time. In the example illustrated in FIG. 2(2), the positional deviation ΔP₀ has occurred between the designated sampling position P₀ and the leading end position Pₜ of the tip TP.

Therefore, firstly, in the sampling system 100-P, there is a problem in that, in sampling at a cell level at which the positional deviation ΔP₀ of about µm has an impact, it is difficult to accurately take the sample SP from the designated sampling position P₀. Secondly, the sampling system 100-P may wait until the temperature T is stabilized to reduce the positional deviation ΔP₀ caused by the temperature T, and therefore, there is a problem in that it is difficult to reduce a time that is needed to take the sample SP.

### 1-3-2. Overview and effects of sampling system 100

The sampling system 100 according to one embodiment performs processes as described below. Firstly, the sampling apparatus 10 acquires the measurement data D that indicates the positional information on each of the tips in use and the temperature T. Secondly, the sampling apparatus 10 inputs, as the training data, the acquired measurement data D to the correction model CM and trains the correction model CM. Thirdly, the sampling apparatus 10, at the start of sampling of the sample SP, identifies each of the storage positions Pₛ in the tip rack TR and the temperature T, and inputs the identified information to the correction model CM to calculate the positional deviation ΔP₀ of each of the tips TP. Fourthly, the sampling apparatus 10 refers to the calculated positional deviation ΔP₀ of each of the tips TP, corrects the leading end position Pₜ of each of the tips TP, and takes the sample SP.

In the sampling system 100 according to one embodiment, following effects are achieved. Firstly, in the sampling system 100, it is possible to reduce an influence of the positional deviation ΔP₀ that is caused by multiple factors and that is complicated, and it is possible to accurately take the sample SP from the designated sampling position P₀. Secondly, in the sampling system 100, it is not needed to wait until the temperature T is stabilized to reduce the positional deviation ΔP₀ that is caused by the temperature T, it is possible to reduce a time needed for sampling of the sample SP.

As described above, in the sampling system 100, it is possible to improve the sampling efficiency of the sample SP.

### 2. Configuration and process of each of apparatuses in sampling system 100

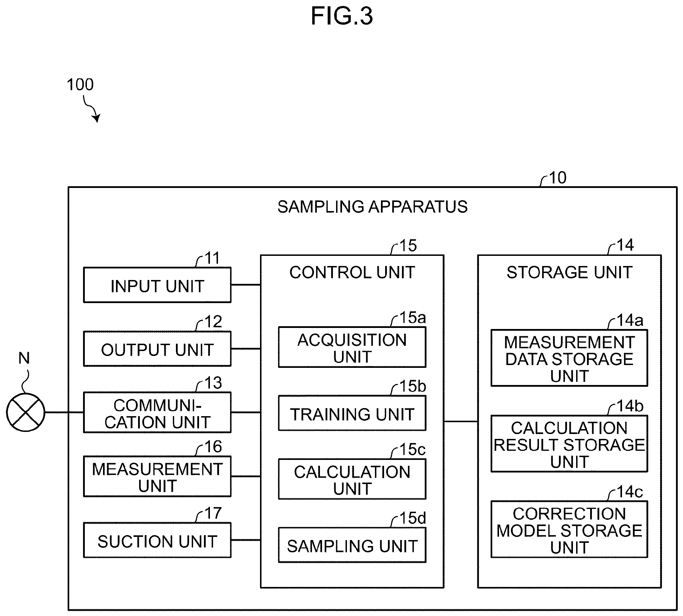

A configuration and a process of each of the apparatuses included in the sampling system 100 will be described below with reference to FIG. 3 to FIG. 6. In the following, a configuration example of the entire sampling system 100 according to one embodiment, and a configuration example and a process example of the sampling apparatus 10 will be described.

### 2-1. Configuration example of entire sampling system 100

A configuration example of the entire sampling system 100 will be described below with reference to FIG. 3. FIG. 3 is a block diagram illustrating a configuration example of each of the apparatuses of the sampling system 100 according to one embodiment. As illustrated in FIG. 3, the sampling system 100 according to one embodiment includes the sampling apparatus 10. Further, the sampling apparatus 10 is communicably connected by a communication network N that is implemented by the Internet or a dedicated line. Further, the sampling system 100 includes the sampling apparatus 10 and a control apparatus (not illustrated) that controls the sampling apparatus 10, and the control apparatus may be communicably connected by the communication network N. In this case, a component corresponding to a control unit 15 of the sampling apparatus 10 is included in the control apparatus.

### 2-2. Configuration example and process example of sampling apparatus 10

A configuration example and a process example of the sampling apparatus 10 will be described below with reference to FIG. 3. The sampling apparatus 10 is a sampling apparatus that takes the sample SP from the designated sampling position P₀ in the analysis target by using the tip TP. Further, the analysis target is, for example, a cell in culture. The sampling apparatus 10 includes an input unit 11, an output unit 12, a communication unit 13, a storage unit 14, the control unit 15, a measurement unit 16, and a suction unit 17.

### 2-2-1. Input unit 11

The input unit 11 controls input of various kinds of information to the sampling apparatus 10. For example, the input unit 11 is implemented by a mouse, a keyboard, or the like, and receives input of various kinds of information to the sampling apparatus 10.

### 2-2-2. Output unit 12

The output unit 12 controls output of various kinds of information from the sampling apparatus 1. For example, the output unit 12 is implemented by a display or the like, and displays various kinds of information stored in the sampling apparatus 10.

### 2-2-3. Communication unit 13

The communication unit 13 controls communication with a different apparatus. For example, the communication unit 13 performs data communication with each of communication apparatuses via a router or the like. Further, the communication unit 13 is able to perform data communication with a terminal or the like (not illustrated).

### 2-2-4. Storage unit 14

The storage unit 14 stores therein various kinds of information that are referred to when the control unit 15 operates, and various kinds of information that are acquired when the control unit 15 operates. The storage unit 14 includes a measurement data storage unit 14a, a calculation result storage unit 14b, and a correction model storage unit 14c. Here, the storage unit 14 may be implemented by, for example, a semiconductor memory device such as a Random Access Memory (RAM) or a flash memory, or a storage device, such as a hard disk or an optical disk. Meanwhile, in the example illustrated in FIG. 3, the storage unit 14 is arranged inside the sampling apparatus 10, but may be arranged outside the sampling apparatus 10 or it may be possible to arrange a plurality of storage units.

### 2-2-4-1. Measurement data storage unit 14a

The measurement data storage unit 14a stores therein the measurement data D. For example, the measurement data storage unit 14a stores therein measurement data D_{A} that is detected by the measurement unit 16 (to be described later), and acquired by an acquisition unit 15a of the control unit 15 (to be described later).

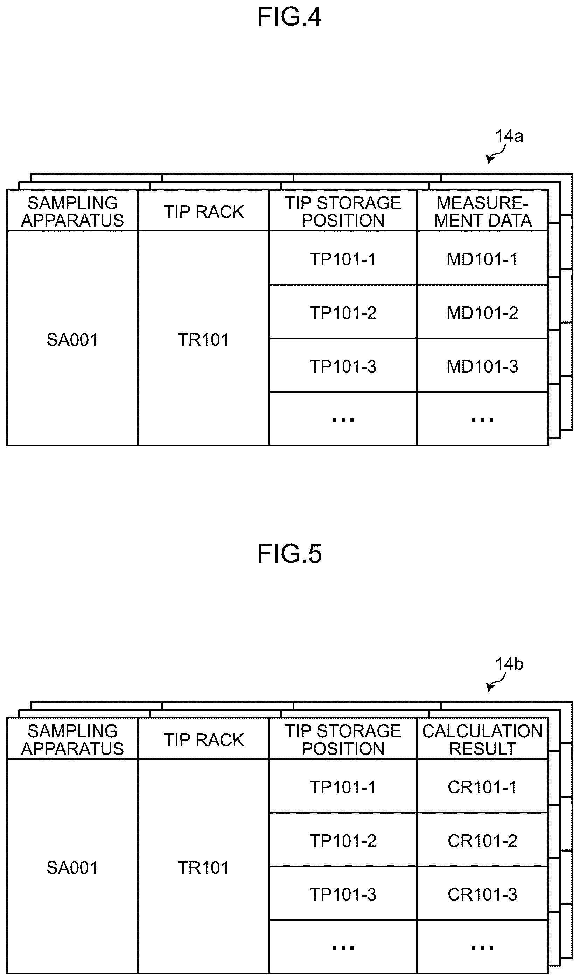

An example of data stored in the measurement data storage unit 14a will be described below with reference to FIG. 4. FIG. 4 is a diagram illustrating an example of the measurement data storage unit 14a of the sampling apparatus 10 according to one embodiment. In the example illustrated in FIG. 4, the measurement data storage unit 14a includes items, such as a "sampling apparatus", a "tip rack", a "tip storage position", and "measurement data".

The "sampling apparatus" indicates identification information for identifying a sampling apparatus that takes the sample SP in an analysis target. For example, the "sampling apparatus" is an identification number or an identification symbol of the sampling apparatus 10 that takes a nucleus, a cell fluid, or a site or a region in a cell from a cell in culture.

The "tip rack" indicates identification information for identifying the tip rack TR for storing the tip TP that is used to take the sample SP. For example, the "tip rack" is an identification number or an identification symbol of the tip rack TR for storing the tip TP that is used by the sampling apparatus 10. Further, the "tip rack" may include, as the identification information on the tip rack TR, a manufacturer name, a model number, a manufacturing date, a material of the tip TP, the number of the tips TP, or the like.

The "tip storage position" indicates identification information for identifying a position of the tip TP that is stored in the tip rack TR. For example, the "tip storage position" is represented by a combination of an alphabet and a number, where the alphabet (for example: A to H) represents a vertical position and a number (for example: 1 to 8) represents a horizontal position in the tip rack TP installed in the sampling apparatus 10. Further, the "tip storage position" is represented by order of use of the sampling apparatus 10.

The "measurement data" is the measurement data D that includes the positional information on each of the tips TP in use. For example, the "measurement data" is positional information on each of the tips TP at each of the storage positions Pₛ in the tip rack TR, with respect to each of the tips TP before use. Further, the "measurement data" is image data D_{I} that includes the image I in which a leading end of each of the tips TP is captured, and indicates time series data of positional information from the time of attachment of each of the tips TP to puncture, for example. Furthermore, for example, the "measurement data" is time series data of the temperature T from the time of attachment of each of the tips TP to puncture. Moreover, the "measurement data" may be time series data of the positional information or the temperature T from the start of use of the sampling apparatus 10 to the end of use of the sampling apparatus 10, and a measurement period is not specifically limited.

Specifically, FIG. 4 illustrates an example in which the measurement data storage unit 14a stores therein, with respect to the sampling apparatus 10 that is identified by "SA001" and the tip rack TR that is identified by "TR101", {tip storage position: "TP101-1", measurement data: "MD101-1"}, {tip storage position: "TP101-2", measurement data: "MD101-2"}, {tip storage position: "TP101-3", measurement data: "MD101-3"}, ... as the measurement data D.

### 2-2-4-2. Calculation result storage unit 14b

The calculation result storage unit 14b stores therein the calculation result CR. For example, the calculation result storage unit 14b stores therein the calculation result CR that is output by a calculation unit 15c of the control unit 15 (to be described later).

An example of data stored in the calculation result storage unit 14b will be described below with reference to FIG. 5. FIG. 5 is a diagram illustrating an example of the calculation result storage unit 14b of the sampling apparatus 10 according to one embodiment. In the example illustrated in FIG. 5, the calculation result storage unit 14b includes items, such as a "sampling apparatus", a "tip rack", a "tip storage position", and a "calculation result". Meanwhile, the items of the "sampling apparatus", the "tip rack", and the "tip storage position" are the same as those of the measurement data storage unit 14a, and therefore, explanation thereof will be omitted.

The "calculation result" is the calculation result CR that is output by the calculation unit 15c based on the measurement data D. For example, the "calculation result" is the positional deviation ΔP₀ that is an error between the designated sampling position P₀ and the leading end position Pₜ of the tip TP, and represented by a combination of a distance (m) that is positional deviation Δx in an X-axis direction and a distance (m) that is positional deviation Δy in a Y-axis direction. Further, the "calculation result" is the positional deviation ΔP₀ that is an error between the designated sampling position P₀ and the leading end position Pₜ of the tip TP, and is represented by, for example, a direction and a straight-line distance (m).

Specifically, FIG. 5 illustrates an example in which the calculation result storage unit 14b stores therein, with respect to the sampling apparatus 10 that is identified by "SA001" and the tip rack TR that is identified by "TR101", {tip storage position: "TP101-1", calculation result: "CR101-1"}, {tip storage position: "TP101-2", calculation result: "CR101-2"}, {tip storage position: "TP101-3", calculation result: "CR101-3"}, ... as the calculation result CR.

### 2-2-4-3. Correction model storage unit 14c

The correction model storage unit 14c stores therein the correction model CM. For example, the correction model storage unit 14c stores therein the correction model CM that is used by the calculation unit 15c of the control unit 15 (to be described later). Further, the correction model storage unit 14c stores therein the correction model CM that is trained by a training unit 15b of the control unit 15 (to be described later).

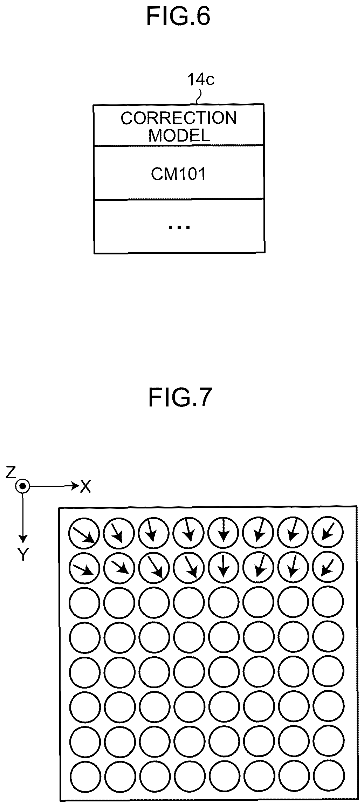

An example of information that is stored in the correction model storage unit 14c will be described below with reference to FIG. 6. FIG. 6 is a diagram illustrating an example of the correction model storage unit 14c of the sampling apparatus 10 according to one embodiment. In the example illustrated in FIG. 6, the correction model storage unit 14c includes an item, such as a "correction model".

The "correction model" is model data of the correction model CM that is a trained machine learning model. For example, the "correction model" is execution data for executing an algorithm of the correction model CM, or data including a model parameter, a hyper parameter, or the like that is a setting value.

FIG. 6 illustrates an example in which the correction model storage unit 14c stores therein a "correction model CM101", ... as the trained correction model CM.

### 2-2-5. Control unit 15

The control unit 15 controls the entire sampling apparatus 10. The control unit 15 includes the acquisition unit 15a, the training unit 15b, the calculation unit 15c, and a sampling unit 15d. Here, the control unit 15 may be implemented by, for example, an electronic circuit, such as a Central Processing Unit (CPU) or a Micro Processing Unit (MPU), or an integrated circuit, such as an Application Specific Integrated Circuit (ASIC) or a Field Programmable Gate Array (FPGA).

### 2-2-5-1. Acquisition unit 15a

The acquisition unit 15a acquires various kinds of information. Meanwhile, the acquisition unit 15a may store the various kinds of acquired information in the storage unit 14. In the following, an acquisition control process will be described.

### Acquisition control process

The acquisition unit 15a performs the acquisition control process. For example, the acquisition unit 15a acquires the measurement data D that includes the positional information on each of the tips TP in use. Further, the acquisition unit 15a acquires the measurement data D that includes the positional information on each of the tips TP at each of the storage positions Pₛ in the tip rack TR that is a storage container for each of the tips TP. Furthermore, the acquisition unit 15a acquires, as the measurement data D including the positional information, the image data D_{I} that includes the image I in which the leading end of each of the tips TP is captured at regular time intervals. Moreover, the acquisition unit 15a acquires, as the measurement data D including the temperature T, the temperature data D_{T} including the temperature T that is detected at regular time intervals.

A specific example of the acquisition control process will be described below. Firstly, the acquisition unit 15a acquires, as the measurement data D of the tip rack TR that is identified by "TR101", {tip storage position: "TP101-1", measurement data: "MD101-1"}, {tip storage position: "TP101-2", measurement data: "MD101-2"}, {tip storage position: "TP101-3", measurement data: "MD101-3"}, ... as the measurement unit 16 of the sampling apparatus 10 that is identified by "SA001". Secondly, the acquisition unit 15a stores the acquired measurement data D in the measurement data storage unit 14a.

Meanwhile, details of the acquisition control process will be described later in Section 3. Specific example of each of processes of sampling system 100 and Section 3-1. Specific example of acquisition control process.

### 2-2-5-2. Training unit 15b

The training unit 15b trains the correction model CM that is a machine learning model. Meanwhile, the training unit 15b may refer to various kinds of information stored in the storage unit 14. In the following, a training control process will be described.

### Training control process

The training unit 15b performs the training control process. For example, the training unit 15b inputs training data that is generated from the measurement data D and that includes each of the storage positions Pₛ of each of the tips TP, the temperature T, the positional deviation ΔP, and the like to the correction model CM, and trains the correction model CM.

A specific example of the training control process will be described below. Firstly, the training unit 15b refers to, with respect to the sampling apparatus 10 that is identified by "SA001" and the tip rack TR that is identified by "TR101", {tip storage position: "TP101-1", measurement data: "MD101-1"}, {tip storage position: "TP101-2", measurement data: "MD101-2"}, {tip storage position: "TP101-3", measurement data: "MD101-3"}, ... as the measurement data D that is stored in the measurement data storage unit 14a. Secondly, the training unit 15b generates, as training data, {tip storage position: "TP101-1", training data: "TD101-1 "}, {tip storage position: "TP101-2", training data: "TD101-2"}, {tip storage position: "TP101-3", training data: "TD101-3"}, .... Thirdly, the training unit 15b inputs the generated training data and trains "CM101" that is the correction model CM stored in the correction model storage unit 14c.

Meanwhile, details of the training control process will be described later in Section 3. Specific example of each of processes of sampling system 100 and Section 3-2. Specific example of training control process.

### 2-2-5-3. Calculation unit 15c

The calculation unit 15c calculates various kinds of information. Meanwhile, the calculation unit 15c may store the output calculation result CR in the storage unit 14. Further, the calculation unit 15c may refer to the various kinds of information stored in the storage unit 14. In the following, a calculation control process will be described.

### Calculation control process

The calculation unit 15c performs the calculation control process. For example, the calculation unit 15c calculates, based on the positional information indicated by the acquired measurement data D, the positional deviation ΔP₀ that is an error with respect to the sampling position P₀ of each of the tips TP. Further, the calculation unit 15c calculates, by using the positional information indicated by the acquired measurement data D, the positional deviation ΔP₀ on a plane of the analysis target with respect to each of the tips TP at each of the storage positions Pₛ. Furthermore, the calculation unit 15c calculates, by using the positional information indicated by the measurement data D corresponding to an elapsed time of each of the tips TP, the positional deviation ΔP₀ of each of the tips TP at the time of puncturing the sample SP. Moreover, the calculation unit 15c calculates, in accordance with the temperature T at the time of attachment of each of the tips TP, the positional deviation ΔP₀ of each of the tips TP at the time of puncturing the sample SP. Furthermore, the calculation unit 15c applies the linear interpolation method to the positional information indicated by the acquired measurement data D, and calculates the positional deviation ΔP₀ of each of the tips TP. Moreover, the calculation unit 15c calculates the positional deviation ΔP₀ of each of the tips TP by using the correction model CM that is subjected to machine learning with a relationship between the positional information indicated by the measurement data D and the positional deviation ΔP₀.

A specific example of the calculation control process will be described below. Firstly, the calculation unit 15c identifies, with respect to the sampling apparatus 10 that is identified by "SA001" and the tip rack TR that is identified by "TR101", each of the storage positions Pₛ of each of the tips TP, the temperature T, and a time t1 at the start of sampling of the sample SP. Secondly, the calculation unit 15c inputs each of the storage positions Pₛ of each of the tips TP, the temperature T, the time t1 that are measured in "CM101" that is the trained correction model CM stored in the correction model storage unit 14c. Thirdly, the calculation unit 15c acquires, as the calculation result CR including the positional deviation ΔP₀ that is output by the correction model CM for each of the tips TP at the time of puncture, {tip storage position: "TP101-1", calculation result: "CR101-1"}, {tip storage position: "TP101-2", calculation result: "CR101-2"}, {tip storage position: "TP101-3", calculation result: "CR101-3"}, .... Fourthly, the calculation unit 15c stores the calculation result CR in the calculation result storage unit 14b.

Meanwhile, details of the calculation control process will be described later in Section 3. Specific example of each of processes of sampling system 100 and Section 3-3. Specific example of calculation control process.

### 2-2-5-4. Sampling unit 15d

The sampling unit 15d performs various kinds of sampling processing. Meanwhile, the sampling unit 15d may refer to various kinds of information stored in the storage unit 14. In the following, a sampling control process will be described.

### Sampling control process

The sampling unit 15d performs the sampling control process. For example, the sampling unit 15d corrects the calculated positional deviation ΔP₀ and takes the sample SP from the analysis target.

A specific example of the sampling control process will be described below. Firstly, the sampling unit 15d refers to, with respect to the sampling apparatus 10 that is identified by "SA001" and the tip rack TR that is identified by "TR101", {tip storage position: "TP101-1", calculation result: "CR101-1"} as the calculation result CR that is stored in the calculation result storage unit 14b. Secondly, the sampling unit 15d attaches the tip TP at the tip storage position of "TP101-1" via the suction unit 17. Thirdly, the sampling unit 15d corrects the leading end position Pₜ of the tip TP by using the calculation result of "CR101-1 ", and punctures the analysis target with the tip TP via the suction unit 17. Fourthly, the sampling unit 15d sucks the sample SP via the suction unit 17. Fifthly, the sampling unit 15d ejects the sample SP to the storage container via the suction unit 17.

Meanwhile, details of the sampling control process will be described later in Section 3. Specific example of each of processes of sampling system 100 and Section 3-4. Specific example of sampling control process.

### 2-2-6. Measurement unit 16

The measurement unit 16 measures various kinds of information. For example, the measurement unit 16 detects the measurement data D including the positional information on each of the tips TP in use. Further, the measurement unit 16 detects, as the measurement data D, the image data D_{I} that includes the image I in which the leading end of each of the tips TP in use is captured, and time series data of positional information from the start of use to sampling of the sample SP, for example, from the time of attachment of the tip TP to the time of puncture with the tip TP. Furthermore, the measurement unit 16 detects, as the measurement data D, the temperature data D_{T} that is obtained by measuring the temperature T of each of the tips TP in use and time series data of the temperature T from the start of use to sampling of the sample SP, for example, from the time of attachment of the tip TP to the time of puncture with the tip TP.

### 2-2-7. Suction unit 17

The suction unit 17 performs various kinds of operation on the tip TP. For example, the suction unit 17 performs, in accordance with a control signal of the sampling unit 15d, operation of attaching the tip TP stored in the tip rack TR, moving the tip TP, puncturing a cell with the tip TP, sucking the sample SP, and ejecting the sample SP to the storage container to take the sample SP.

### 3. Specific example of each of processes of sampling system 100

A specific example of each of processes of the sampling system 100 according to one embodiment will be described. In the following, a specific example of the acquisition control process, a specific example of the training control process, a specific example of the calculation control process, and a specific example of the sampling control process will be described.

### 3-1. Specific example of acquisition control process

A specific example of the acquisition control process will be described. In the following, a specific example of an image data acquisition control process, a specific example of a temperature data acquisition control process, and a specific example of a various data acquisition control process.

### 3-1-1. Specific example of image data acquisition control process

A specific example of the image data acquisition control process will be described below with reference to FIG. 7 to FIG. 9. For example, the sampling apparatus 10 detects, as the image data D_{I}, the image I that is captured at regular time intervals via the measurement unit 16, such as an imaging apparatus. In this case, the sampling apparatus 10 captures, in a time lapse manner, the image I that is enlarged by a confocal microscope that is installed below a culture container in which a cell as the analysis target is stored. Further, the sampling apparatus 10 outputs the positional deviation ΔP that is an error with respect to a movement path, from the image I corresponding to an elapsed time Δt from the time t1 to the time t2 at which the leading end of the tip TP is captured. Here, the sampling apparatus 10 acquires, for example, time series data of the positional deviation ΔP for the elapsed time Δt from the time t1 at which the tip TP is attached to the time t2 at which puncture is performed with the tip TP. Meanwhile, the start of measurement of the time series data of the positional deviation ΔP may be the start of use of the sampling apparatus 10. Further, the end of measurement of the time series data of the positional deviation ΔP may be the time of suction or ejection of the sample SP or the end of use of the sampling apparatus 10.

The sampling apparatus 10 repeats the above-described processes on all of the storage positions Pₛ in the tip rack TR. Further, the sampling apparatus 10 may acquire the measurement data D at the time of actually taking the sample SP, or may acquire the measurement data D at the time of trial operation in which the sample SP is not taken. Further, the sampling apparatus 10 may acquire measurement data D' that is output by a different sampling apparatus 10'. Furthermore, the sampling apparatus 10 may acquire the measurement data D for each type (for example: a manufacturer, a model number, a material, or the number) of the tip rack TR.

A specific example 1 of the image data D_{I} will be described with reference to FIG. 7. FIG. 7 is a diagram illustrating the specific example 1 of the measurement data D according to one embodiment. For example, the image data D_{I} is acquired at each of the storage positions Pₛ of the tip rack TR. In the example illustrated in FIG. 7, the tip rack TR has the storage position Pₛ for the tips TP at a total of 64 positions of a matrix of 8 by 8 arranged on a XY plane. Here, assuming that each of the positions in a positive Y-axis direction is represented by an alphabet from A to H, and each of the positions in a positive X-axis direction is represented by a number from 1 to 8, the storage position Pₛ at the upper left is represented by "A1". Furthermore, in FIG. 7, an arrow in each of the storage positions Pₛ indicates an image of a direction and a size of the positional deviation ΔP; for example, it is indicated that positional deviation ΔP_{A1} of "A1" occurs toward a southeast side (lower right side) and positional deviation ΔP_{B8} of "B8" occurs toward a southwest side (lower left side). Meanwhile, illustration of arrows at the storage positions Pₛ from "C" to "H" is omitted.

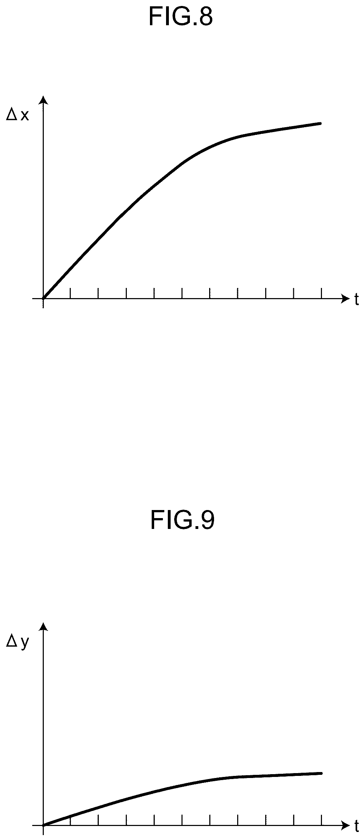

A specific example 2 of the image data D_{I} will be described with reference to FIG. 8. FIG. 8 is a diagram illustrating the specific example 2 of the measurement data D according to one embodiment. For example, the image data D_{I} is acquired as time series data of the positional deviation Δx in the X-axis direction with respect to each of the storage positions Pₛ in the tip rack TR. In the example illustrated in FIG. 8, at the specific storage position Pₛ (for example: "A1"), the positional deviation Δx in the X-axis direction increases over time. Here, the image data D_{I} is acquired as, for example, the time series data of the positional deviation Δx in the X-axis direction from the time of attachment of the tip TP to the time of puncture with the tip TP.

A specific example 3 of the image data D_{I} will be described with reference to FIG. 9. FIG. 9 is a diagram illustrating the specific example 3 of the measurement data D according to one embodiment. For example, the image data D_{I} is acquired as the time series data of the positional deviation Δy in the Y-axis direction with respect to each of the storage positions Pₛ in the tip rack TR. In the example illustrated in FIG. 9, at the specific storage position Pₛ (for example: "A1"), the positional deviation Δy in the Y-axis direction increases over time. Here, the image data D_{I} is acquired as, for example, the time series data of the positional deviation Δy in the Y-axis direction from the time of attachment of the tip TP to the time of puncture with the tip TP.

### 3-1-2. Specific example of temperature data acquisition control process

A specific example of the temperature data acquisition control process will be described. For example, the sampling apparatus 10 detects, as the temperature data D_{T}, the temperature T inside the sampling apparatus 10, which is measured at regular time intervals via the measurement unit 16, such as a temperature sensor. Here, the temperature data D_{T} is acquired as, for example, time series data of the temperature T from the time of attachment of the tip TP to the time of puncture with the tip TP.

### 3-1-3. Specific example of various data acquisition control process

A specific example of the various data acquisition control process will be described. For example, the sampling apparatus 10 may detect, as temperature data D_{T}', temperature T' outside the sampling apparatus 10, which is measured at regular time intervals via the measurement unit 16, such as a temperature sensor. Further, the sampling apparatus 10 may detect, as vibration data D_{V}, the vibration V inside and outside the sampling apparatus 10, which is measured at regular time intervals via the measurement unit 16, such as a vibration sensor. Furthermore, the sampling apparatus 10 may detect, as positional data D_{Z}, a position Z of the tip TP in a Z-axis direction, which is measured at regular time intervals via the measurement unit 16, such as a laser device.

### 3-2. Specific example of training control process

A specific example of the training control process will be described. Firstly, the sampling apparatus 10 inputs, as the training data, {storage position: "A1", time: "t1", positional deviation Δx in X-axis direction: "Δx_{A1}(t1)", positional deviation Δy in Y-axis direction : "Δy_{A1}(t1)", temperature: "T_{A1}"}, {storage position: "A2", time: "t1", positional deviation Δx in X-axis direction: "Δx_{A2}(t1)", positional deviation Δy in Y-axis direction : "Δy_{A2}(t1)", temperature: "T_{A2}"}, {storage position: "A3", time: "t1", positional deviation Δx in X-axis direction: "Δx_{A3}(t1)", positional deviation Δy in Y-axis direction : "Δy_{A3}(t1)", temperature: "T_{A3}"}, ... to the correction model CM. Secondly, the sampling apparatus 10 evaluates the positional deviation ΔP₀ that is output from the correction model CM by using evaluation data. Thirdly, the sampling apparatus 10 updates a hyper parameter of the correction model CM in accordance with an evaluation result.

Meanwhile, the sampling apparatus 10 may train the correction model CM by using training data that includes the temperature data D_{T}' indicating the temperature T' outside the sampling apparatus 10, the vibration data D_{V} indicating the vibration V inside and outside the sampling apparatus 10, and the positional data D_{Z} indicating the position Z of the tip TP in the Z-axis direction.

### 3-3. Specific example of calculation control process

A specific example of the calculation control process will be described. In the following, a specific example of a linear interpolation calculation process and a specific example of a correction model calculation process will be described.

### 3-3-1. Specific example of linear interpolation calculation process

A specific example of the linear interpolation calculation process will be described. Firstly, the sampling apparatus 10, at the start of sampling of the sample SP, identifies each of the storage positions Pₛ and identifies the time t1 and the temperature T at the time of attachment of the tip TP. Secondly, the sampling apparatus 10 refers to, with respect to each of the storage positions Pₛ, the measurement data D in which the time t1, the temperature T, the positional deviation Δx in the X-axis direction, and the positional deviation Δy in the Y-axis direction are plotted. Thirdly, the sampling apparatus 10 outputs the calculation result CR by applying, with respect to the identified storage position Pₛ, the positional deviation Δx in the X-axis direction and the positional deviation Δy in the Y-axis direction at the time T2 at the time of puncture with the tip TP and corresponding to the time t1 and the temperature T to the linear interpolation method. In other words, the sampling apparatus 10 is able to calculate the positional deviation Δx in the X-axis direction and the positional deviation Δy in the Y-axis direction, that is, the positional deviation ΔP₀ by taking into account the elapsed time Δt from the time of attachment of the tip TP to the time of puncture of the sample SP.

Meanwhile, the sampling apparatus 10 is able to calculate the positional deviation ΔP₀ by further using the temperature T' outside the sampling apparatus 10 and the vibration V inside and outside the sampling apparatus 10. Further, the sampling apparatus 10 is able to output positional deviation Δz of the tip TP in the Z-axis direction as the calculation result CR. Furthermore, the sampling apparatus 10 is able to output the calculation result CR for each type (for example: a manufacturer, a model number, a material, and the number) of the tip rack TR.

### 3-3-2. Specific example of correction model calculation process

A specific example of the correction model calculation process will be described. Firstly, the sampling apparatus 10 identifies each of the storage positions Pₛ at the start of sampling of the sample SP, and identifies the time t1 and the temperature T at the time of attachment of the tip TP. Secondly, the sampling apparatus 10 inputs each of the identified storage positions Pₛ, the identified time t1, and the identified temperature T to the trained correction model CM. Thirdly, the sampling apparatus 10 acquires, as the calculation result CR, the positional deviation Δx in the X-axis direction and the positional deviation Δy in the Y-axis direction at the time t2 at the time of puncturing the sample SP, which is output by the trained correction model CM. In other words, the sampling apparatus 10 is able to calculate the positional deviation Δx in the X-axis direction and the positional deviation Δy in the Y-axis direction, that is, the positional deviation ΔP₀, by taking into account the elapsed time Δt from the time t1 at the time of attachment of the tip TP to the time t2 at the time of puncturing the sample SP.

Meanwhile, the sampling apparatus 10 is able to calculate the positional deviation ΔP₀ by further inputting the temperature T' outside the sampling apparatus 10 and the vibration V inside and outside the sampling apparatus 10. Further, the sampling apparatus 10 is able to output, as the calculation result CR, the positional deviation Δz of the tip TP in the Z-axis direction. Furthermore, the sampling apparatus 10 is able to output the calculation result CR for each type (for example: a manufacturer, a model number, a material, and the number) of the tip rack TR.

### 3-4. Specific example of sampling control process

A specific example of the sampling control process will be described. Firstly, the sampling apparatus 10 refers to the positional deviation Δx in the X-axis direction and the positional deviation Δy in the Y-axis direction indicated by the calculation result CR with respect to the identified storage position Pₛ. Secondly, the sampling apparatus 10 corrects the leading end position Pₜ of the tip TP by using the positional deviation Δx in the X-axis direction and the positional deviation Δy in the Y-axis direction. Thirdly, the sampling apparatus 10 attaches the tip TP, moves the tip TP to the corrected leading end position Pₜ, inserts the tip TP, sucks the sample SP, and ejects the sample SP to the storage container via the suction unit 17, so that the sample SP is taken.

Meanwhile, the sampling apparatus 10 is able to correct the positional deviation Δz of the tip TP in the Z-axis direction and take the sample SP. Further, the sampling apparatus 10 is able to cancel sampling of the sample SP when it is difficult to correct the leading end position Pₜ of the tip TP.

### 4. Flow of each of processes of sampling system 100

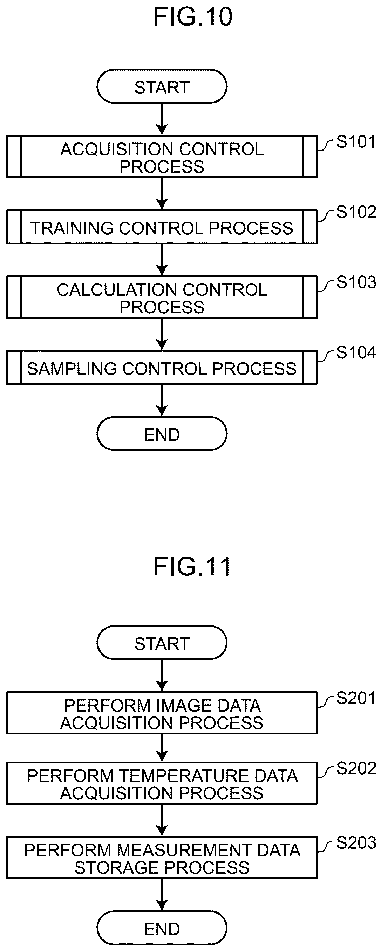

A flow of processes performed by the sampling system 100 according to one embodiment will be described with reference to FIG. 10 to FIG. 14. In the following, a flow of the entire process of the sampling system 100 will be first described, and thereafter, as each of the processes, a flow of the acquisition control process, a flow of the training control process, a flow of the calculation control process, and a flow of the sampling control process will be described.

### 4-1. Flow of entire process of sampling system 100

The flow of the entire process of the sampling system 100 according to one embodiment will be described with reference to FIG. 10. FIG. 10 is a flowchart illustrating an example of the flow of the entire process of the sampling system 100 according to one embodiment. Meanwhile, processes from Step S101 to S104 below may be performed in different order. Further, some of the processes from Step S101 to S104 below may be omitted.

### 4-1-1. Acquisition control process

Firstly, the sampling system 100 performs the acquisition control process (Step S101). For example, the sampling system 100 performs processes from Step S201 to Step S203 (to be described later), and controls acquisition of the measurement data D including the positional information on each of the tips TP.

### 4-1-2. Training control process

Secondly, the sampling system 100 performs the training control process (Step S102). For example, the sampling system 100 performs processes from Step S301 to Step S304 (to be described later), and controls training of the correction model CM that is a machine learning model.

### 4-1-3. Calculation control process

Thirdly, the sampling system 100 performs the calculation control process (Step S103). For example, the sampling system 100 performs processes from Step S401 to Step S405 (to be described later), and controls calculation of the positional deviation ΔP₀.

### 4-1-4. Sampling control process

Fourthly, the sampling system 100 performs the sampling control process (Step S104), and terminates the entire process. For example, the sampling system 100 performs processes from Step S501 to Step S505 (to be described later), and controls sampling of the sample SP.

### 4-2. Flow of acquisition control process

The flow of the acquisition control process of the sampling system 100 according to one embodiment will be described with reference to FIG. 11. FIG. 11 is a flowchart illustrating an example of the flow of the acquisition control process of the sampling system 100 according to one embodiment. Meanwhile, the processes from Step S201 to Step S203 below may be performed in different order. Further, some of the processes from Step S201 to Step S203 below may be omitted.

### 4-2-1. Image data acquisition process

Firstly, the sampling apparatus 10 performs an image data acquisition process (Step S201). For example, the sampling apparatus 10 acquires the image data D_{I} that includes the image I that is captured at regular time intervals by the measurement unit 16, such as an imaging apparatus.

### 4-2-2. Temperature data acquisition process

Secondly, the sampling apparatus 10 performs a temperature data acquisition process (Step S202). For example, the sampling apparatus 10 acquires the temperature data D_{T} that includes the temperature T that is detected at regular time intervals by the measurement unit 16, such as a temperature sensor.

### 4-2-3. Measurement data storage process

Thirdly, the sampling apparatus 10 performs a measurement data storage process (Step S203), and terminates the acquisition control process. For example, the sampling apparatus 10 stores the acquired image data D_{I} and the acquired temperature data D_{T} in the measurement data storage unit 14a.

### 4-3. Flow of training control process

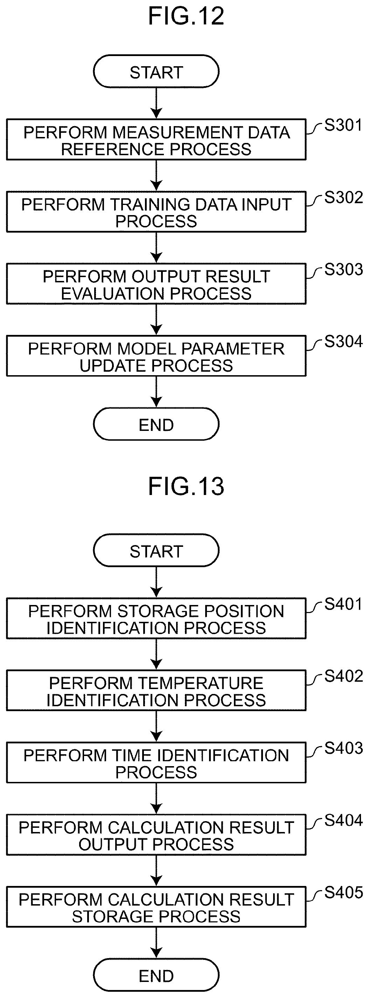

The flow of the training control process of the sampling system 100 according to one embodiment will be described with reference to FIG. 12. FIG. 12 is a flowchart illustrating an example of the flow of the training control process of the sampling system 100 according to one embodiment. Meanwhile, the processes from Step S301 to Step S304 below may be performed in different order. Further, some of the processes from Step S301 to Step S304 below may be omitted.

### 4-3-1. Measurement data reference process

Firstly, the sampling apparatus 10 performs a measurement data reference process (Step S301). For example, the sampling apparatus 10 refers to the image data D_{I} or the temperature data D_{T} stored in the measurement data storage unit 14a.

### 4-3-2. Training data input process

Secondly, the sampling apparatus 10 performs a training data input process (Step S302). For example, the sampling apparatus 10 generates training data from the image data D_{I} or the temperature data D_{T} and inputs the training data to the correction model CM.

### 4-3-3. Output result evaluation process

Thirdly, the sampling apparatus 10 performs an output result evaluation process (Step S303). For example, the sampling apparatus 10 generates evaluation data from the image data D_{I} or the temperature data D_{T}, inputs the evaluation data to the correction model CM, and evaluates an output result.

### 4-3-4. Model parameter update process

Fourthly, the sampling apparatus 10 performs a model parameter update process (Step S304), and terminates a training control process. For example, the sampling apparatus 10 updates the hyper parameter of the correction model CM in accordance with the evaluation result.

### 4-4. Flow of calculation control process

The flow of the calculation control process of the sampling system 100 according to one embodiment will be described with reference to FIG. 13. FIG. 13 is a flowchart illustrating an example of the flow of the calculation control process of the sampling system 100 according to one embodiment. Meanwhile, the processes from Step S401 to Step S405 below may be performed in different order. Further, some of the processes from Step S401 to Step S405 below may be omitted.

### 4-4-1. Storage position identification process

Firstly, the sampling apparatus 10 performs a storage position identification process (Step S401). For example, the sampling apparatus 10 identifies each of the storage positions Pₛ of each of the tips TP to be used at the start of sampling of the sample SP.

### 4-4-2. Temperature identification process

Secondly, the sampling apparatus 10 performs a temperature identification process (Step S402). For example, the sampling apparatus 10 identifies the temperature T at the time of attachment of each of the tips TP at the start of sampling of the sample SP.

### 4-4-3. Time identification process

Thirdly, the sampling apparatus 10 performs a time identification process (Step S403). For example, at the start of sampling of the sample SP, the sampling apparatus 10 identifies the time t1 at the time of attachment of each of the tips TP.

### 4-4-4. Calculation result output process

Fourthly, the sampling apparatus 10 performs the calculation result output process (Step S404). For example, the sampling apparatus 10 inputs each of the identifies storage positions Pₛ, the identified temperature T, and the identified time t1 to the correction model CM, and calculates the positional deviation ΔP₀ at the time t2 at the time of puncture with each of the tips TP. Further, the sampling apparatus 10 applies the linear interpolation method to each of the identified storage positions Pₛ, the identified temperature T, the identified time t1, and the measurement data D, and calculates the positional deviation ΔP₀ at the time t2 at the time of puncture with each of the tips TP.

### 4-4-5. Calculation result storage process

Fifthly, the sampling apparatus 10 performs a calculation result storage process (Step S405), and terminates the calculation control process. For example, the sampling apparatus 10 stores the calculation result CR that includes the calculated positional deviation ΔP₀ at the time t2 at the time of puncture with each of the tips TP in the calculation result storage unit 14b.

### 4-5. Flow of sampling control process

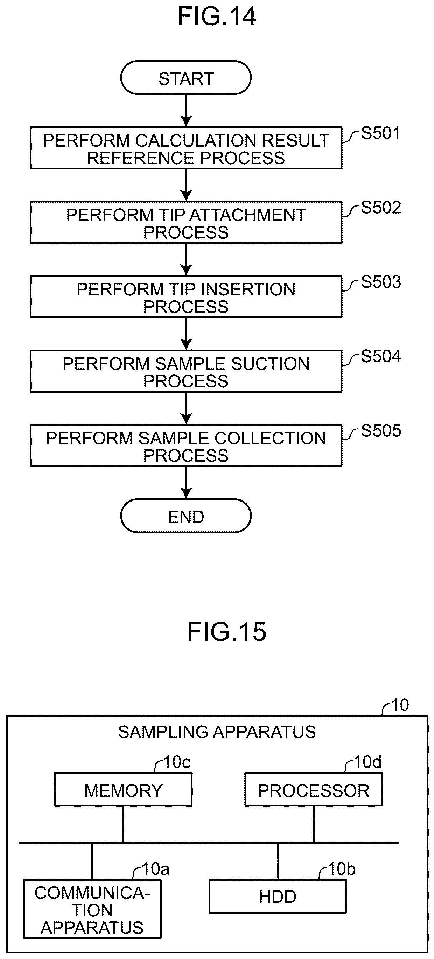

The flow of the sampling control process of the sampling system 100 according to one embodiment will be described with reference to FIG. 14. FIG. 14 is a flowchart illustrating an example of the flow of the sampling control process of the sampling system 100 according to one embodiment. Meanwhile, the processes from Step S501 to Step S505 below may be performed in different order. Further, some of the processes from Step S501 to Step S505 below may be omitted.

### 4-5-1. Calculation result reference process

Firstly, the sampling apparatus 10 performs a calculation result reference process (Step S501). For example, the sampling apparatus 10 refers to the calculation result CR that includes the calculated positional deviation ΔP₀ at the time t2 at the time of puncture with each of the tips TP and that is stored in the calculation result storage unit 14b.

### 4-5-2. Tip attachment process

Secondly, the sampling apparatus 10 performs a tip attachment process (Step S502). For example, the sampling apparatus 10 attaches the tip TP in the tip rack TR via the suction unit 17.

### 4-5-3. Tip insertion process

Thirdly, the sampling apparatus 10 performs a tip insertion process (Step S503). For example, the sampling apparatus 10 corrects the leading end position Pₜ by using the positional deviation ΔP₀ at the time t2 at the time of puncture with the tip TP in accordance with the storage position Pₛ, and punctures a cell in culture that is an analysis target with the leading end of the tip TP via the suction unit 17.

### 4-5-4. Sample suction process

Fourthly, the sampling apparatus 10 performs a sample suction process (Step S504). For example, the sampling apparatus 10 sucks the sample SP in the tip TP via the suction unit 17.

### 4-5-5. Sample collection process

Fifthly, the sampling apparatus 10 performs a sample collection process (Step S505), and terminates the sampling control process. For example, the sample SP is ejected to the storage container via the suction unit 17.

### 5. Effects of one embodiment

Effects of one embodiment will be described below. In the following, a first effect to an eighth effect corresponding to the processes according to one embodiment will be described.

### 5-1. First effect

Firstly, in the process according to one embodiment as described above, the sampling apparatus 10 is a sampling apparatus that takes the sample SP from the designated sampling position P₀ of the analysis target by using the tip TP, acquires the measurement data D including the positional information on each of the tips TP in use, and calculates the positional deviation ΔP₀ that is an error with respect to the sampling position P₀ of each of the tips TP based on the acquired measurement data D. Therefore, in one embodiment, it is possible to improve the sampling efficiency of the sample SP.

### 5-2. Second effect

Secondly, in the process according to one embodiment as described above, the sampling apparatus 10 acquires the measurement data D of each of the tips TP at each of the storage positions Pₛ in the tip rack TR of each of the tips TP, calculates the positional deviation ΔP₀ on a plane of the analysis target with respect to each of the tips TP at each of the storage positions Pₛ by using the acquired measurement data D, corrects the calculated positional deviation ΔP₀, and takes the sample SP from the analysis target. Therefore, in one embodiment, it is possible to accurately take the sample SP from the designated sampling position P₀, so that it is possible to improve the sampling efficiency of the sample SP.

### 5-3. Third effect

Thirdly, in the process according to one embodiment as described above, the sampling apparatus 10 calculates the positional deviation ΔP₀ at the time of puncturing the sample SP with each of the tips TP by using the measurement data D corresponding to an elapsed time of each of the tips TP. Therefore, in one embodiment, it is possible to correct the positional deviation ΔP₀ by taking into account the elapsed time Δt from the time of attachment of the tip TP to the time of puncture, so that it is possible to improve the sampling efficiency of the sample SP.

### 5-4. Fourth effect

Fourthly, in the process according to one embodiment as described above, the sampling apparatus 10 calculates the positional deviation ΔP₀ at the time of puncturing the sample SP with each of the tips TP in accordance with the temperature T at the time of attachment of each of the tips TP. Therefore, in one embodiment, it is possible to correct the positional deviation ΔP₀ by taking into account the temperature T at the time of use of the tip TP, so that it is possible to improve the sampling efficiency of the sample SP.

### 5-5. Fifth effect

Fifthly, in the process according to one embodiment as described above, the sampling apparatus 10 captures, as the measurement data D, the image data D_{I} including the image I in which a leading end of each of the tips TP is captured at regular time intervals. Therefore, in one embodiment, it is possible to use the captured image I as the positional information, so that it is possible to improve the sampling efficiency of the sample SP.

### 5-6. Sixth effect

Sixthly, in the process according to one embodiment as described above, the sampling apparatus 10 applies the linear interpolation method to the acquired measurement data D to calculate the positional deviation ΔP₀ of each of the tips. Therefore, in one embodiment, it is possible to calculate the positional deviation ΔP₀ by linear interpolation, so that it is possible to improve the sampling efficiency of the sample SP.

### 5-7. Seventh effect

Seventhly, in the process according to one embodiment as described above, the sampling apparatus 10 calculates the positional deviation ΔP₀ of each of the tips TP by using the correction model CM that is subjected to machine learning with respect to a relationship between the measurement data D and the positional deviation ΔP₀. Therefore, in one embodiment, it is possible to calculate the positional deviation ΔP₀ by the correction model CM, so that it is possible to improve the sampling efficiency of the sample SP.

### 5-8. Eighth effect

Eighthly, in the process according to one embodiment as described above, the analysis target is a cell in culture. Therefore, in one embodiment, it is possible to improve the sampling efficiency of the sample SP from a cell in culture.

### 6. System

The processing procedures, control procedures, specific names, and information including various kinds of data and parameters illustrated in the above-described document and drawings may be arbitrarily changed unless otherwise specified.

Furthermore, the components of the apparatuses illustrated in the drawings are functionally conceptual and do not necessarily have to be physically configured in the manner illustrated in the drawings. In other words, specific forms of distribution and integration of the apparatuses are not limited to those illustrated in the drawings. That is, all or part of the apparatuses may be functionally or physically distributed or integrated in arbitrary units depending on various loads or use conditions.

Moreover, all or an arbitrary part of various kinds of processing functions that are implemented by the apparatuses may be realized by a CPU or a program that is analyzed and executed by the CPU, or may be realized by hardware using wired logic.

### 7. Hardware

A hardware configuration example of the sampling apparatus 10 will be described below. Meanwhile, the other apparatuses may have the same hardware configurations. FIG. 15 is a diagram illustrating a hardware configuration example according to one embodiment. As illustrated in FIG. 15, the sampling apparatus 10 includes a communication apparatus 10a, a Hard Disk Drive (HDD) 10b, a memory 10c, and a processor 10d. Further, all of the units illustrated in FIG. 15 are connected to one another via a bus or the like.

The communication apparatus 10a is a network interface card or the like, and performs communication with a different server. The HDD 10b stores therein a program or a database for implementing the functions as illustrated in FIG. 3.

The processor 10d reads, from the HDD 10b or the like, a program that executes the same processes as those of each of the processing units illustrated in FIG. 3, loads the program onto the memory 10c, and executes the processes that implement each of the functions illustrated in FIG. 3. For example, the processes implement the same functions as those of each of the processing units that are included in the sampling apparatus 10. Specifically, the processor 10d reads, from the HDD 10b or the like, a program that has the same functions as those of the acquisition unit 15a, the training unit 15b, the calculation unit 15c, the sampling unit 15d, and the like. Further, the processor 10d executes a process for implementing the same processes as those of the acquisition unit 15a, the training unit 15b, the calculation unit 15c, the sampling unit 15d, and the like.

In this manner, the sampling apparatus 10 operates as an apparatus that reads and executes the program to implement various kinds of processing methods. Further, the sampling apparatus 10 is able to implement the same functions as those of the embodiments as described above by causing a medium reader to read the above-described program from a recording medium and executing the above-described read program. Meanwhile, the program described in one embodiment need not always be executed by the sampling apparatus 10. For example, the present disclosure may be applied in the same manner even when a different computer or a different server apparatus executes the above-described program or the different computer and the different server apparatus execute the above-described program in a cooperative manner.

The program according to one embodiment may be distributed via a network, such as the Internet. Further, the program may be recorded in a computer readable recording medium, such as a hard disk, a flexible disk (FD), a compact disc (CD)-ROM, a MagnetoOptical disk (MO), or a Digital Versatile Disk (DVD), and may be executed by being read from the recording medium by a computer.

According to one aspect of the present disclosure, it is possible to improve sampling efficiency.

## Claims

1. A sampling apparatus (10) that takes a sample from a designated sampling position of an analysis target by using a tip, the sampling apparatus comprising:
a processor that
acquires positional information on each of tips in use, and
calculates an error with respect to the sampling position of each of the tips based on the acquired positional information.

2. The sampling apparatus (10) according to claim 1, wherein
the processor
acquires the positional information on each of the tips at each of storage positions in a storage container of each of the tips,
calculates the error on a plane of the analysis target with respect to each of the tips at each of the storage positions by using the acquired positional information,
corrects the calculated error, and
takes the sample from the analysis target.

3. The sampling apparatus (10) according to claim 2, wherein the processor calculates the error at time of puncturing the sample with each of the tips by using the positional information corresponding to an elapsed time of each of the tips.

4. The sampling apparatus (10) according to claim 3, wherein the processor calculates the error at time of puncturing the sample with each of the tips in accordance with temperature at time of attachment of each of the tips.

5. The sampling apparatus (10) according to any one of claims 1 to 4, wherein the processor acquires, as the positional information, an image in which a leading end of each of the tips is captured at regular time intervals.

6. The sampling apparatus according to any one of claims 1 to 5, wherein the processor applies a linear interpolation method to the acquired positional information to calculate the error of each of the tips.

7. The sampling apparatus according to any one of claims 1 to 6, wherein the processor calculates the error of each of the tips by using a machine learning model that is subjected to machine learning with respect to a relationship between the positional information and the error.

8. The sampling apparatus according to any one of claims 1 to 7, wherein the analysis target is a cell in culture.

9. A sampling method implemented by a sampling apparatus (10) that takes a sample from a designated sampling position of an analysis target by using a tip, the sampling method comprising:
acquiring positional information on each of the tips in use; and
calculating an error with respect to the sampling position of each of the tips based on the acquired positional information.

10. A sampling program that causes a sampling apparatus (10) that takes a sample from a designated sampling position of an analysis target by using a tip to execute a process, the process comprising:
acquiring positional information on each of the tips in use; and
calculating an error with respect to the sampling position of each of the tips based on the acquired positional information.
